# DEMANDE DE BREVET EUROPEEN

(11) **EP 1 428 500 A1**
(43) Date de publication de la demande: **16.06.2004**
(21) Numéro de dépôt: 03293060.4
(22) Date de dépôt: 08.12.2003
(51) Int. Cl.: A61K 7/09, A61K 7/13, A61K 7/135

(54) **Composition oxydantes contenant un mélange de polymères dont au moins un copolymère à base d'acrylamide et d'acide 2-acrylamido-2-méthylpropane sulfonique**

(30) Priorité: 09.12.2002 FR 0215546
(71) Demandeur: L'OREAL, 75008 Paris (FR)
(72) Inventeur: Legrand, Frédéric, 92400 Courbevoie (FR); Kravtchenko, Sylvain, 92600 Asnières (FR)
(74) Mandataire: Dossmann, Gérard

(57) **Abrégé**

L'invention concerne une composition oxydante pour fibres kératiniques, en particulier pour fibres kératiniques humaines et plus particulièrement des cheveux, comprenant dans un milieu cosmétiquement acceptable :
- au moins un agent oxydant choisi dans le groupe formé par le peroxyde d'hydrogène et les composés susceptibles de produire par hydrolyse du peroxyde d'hydrogène ou leurs mélanges,
- au moins un copolymère (b) à base d'acide 2-acrylamido-2-méthylpropane sulfonique et d'acrylamide, et
- au moins un polymère (c) choisi parmi les homopolymères d'acide 2-acrylamido-2-méthylpropane sulfonique réticulés ou les copolymères amphiphiles constitués d'au moins une séquence de motifs acide 2-acrylamido-2-méthylpropane sulfonique, et au moins un motif comportant une partie hydrophobe.

L'invention concerne également les procédés et dispositifs de teinture, de permanente, de décoloration et de décapage mettant en oeuvre ladite composition.

## Description

La présente invention a trait à des compositions oxydantes, destinées au traitement des matières kératiniques comprenant un mélange de polymère dont au moins un copolymère à base d'acide 2-acrylamido-2-méthylpropane sulfonique et d'acrylamide, leurs utilisations pour la teinture, pour la déformation permanente ou pour la décoloration ou le décapage des fibres kératiniques humaines et en particulier des cheveux.

Il est connu de décolorer les fibres kératiniques et en particulier les cheveux humains avec des compositions de décoloration contenant un ou plusieurs agents oxydants. Parmi les agents oxydants classiquement utilisés, on peut citer le peroxyde d'hydrogène ou des composés susceptibles de produire par hydrolyse du peroxyde d'hydrogène, tels que le peroxyde d'urée ou les persels comme les perborates, les percarbonates et les persulfates, le peroxyde d'hydrogène étant particulièrement préféré.

Ces compositions de décoloration se présentent généralement sous forme de produits anhydres (poudres ou crèmes) contenant des composés alcalins (amines et silicates alcalins), et un réactif peroxygéné tels qu'un persulfate, perborate ou percarbonate, d'ammonium ou de métaux alcalins, que l'on dilue au moment de l'emploi avec une composition aqueuse de peroxyde d'hydrogène.

Les compositions de décoloration peuvent aussi résulter du mélange, au moment de l'emploi, de la poudre anhydre de réactif péroxygéné avec une composition aqueuse contenant les composés alcalins et une autre composition aqueuse contenant le peroxyde d'hydrogène.

Il est connu par ailleurs de teindre les fibres kératiniques et en particulier les cheveux humains avec des compositions tinctoriales contenant des précurseurs de colorant d'oxydation, en particulier des ortho ou paraphénylène diamines, des ortho ou paraaminophénols, des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés ou colorants. On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

Pour localiser le produit de décoloration ou de teinture sur les cheveux afin qu'il ne coule pas sur le visage ou en dehors des zones que l'on se propose de décolorer, on a jusqu'ici eu recours à l'emploi d'épaississants traditionnels tels que l'acide polyacrylique réticulé, les hydroxyéthylcelluloses, certains polyuréthanes, les cires, et en outre, dans le cas des compositions aqueuses de décoloration, à des mélanges d'agents tensio-actifs ioniques de HLB (Hydrophilic Lipophilic Balance), qui convenablement choisis, engendrent l'effet gélifiant quand on les dilue au moyen d'eau et/ou d'agents tensioactifs.

Plus récemment, on a découvert l'utilisation de polymères amphiphiles comportant au moins un monomère à insaturation éthylènique à groupement sulfonique et plus particulièrement les polymères ou copolymères d'acide acrylamido-2-méthylpropane sulfonique, faisant l'objet de la demande de brevet FR 2 818 537, pour obtenir des solutions de peroxyde d'hydrogène épaissies ou sous forme de gel, stables à la conservation.

Cependant, les formules ainsi épaissies, ou gélifiées, posent un problème d'utilisation lors du mélange de l'oxydant avec un support de coloration. En effet, il se produit dès les premiers instants du mélange une fluidification très importante des deux formules. Même si cette phase de fluidification n'est que transitoire, l'utilisation de ces formules oxydantes est problématique, tout particulièrement pour les professionnels.

La demanderesse a découvert de manière surprenante qu'il est possible de réduire très fortement cette fluidification observée lors des mélanges, en combinant au moins un copolymère à base d'acrylamide et d'acide 2-acrylamido-2-méthyl propane sulfonique à des formules oxydantes contenant au moins un polymère ayant au moins une séquence de motifs acide 2-acrylamido-2-méthyl propane sulfonique particulier. La demanderesse a ainsi obtenu des compositions oxydantes épaissies, stables au stockage, quelle que soit la source en eau oxygénée utilisée et en s'affranchissant des problèmes de fluidification, en particulier en cas de mélange.

L'invention concerne également les procédés de teinture d'oxydation des fibres kératiniques, les procédés de traitement desdites fibres, et en particulier de permanente, des procédés de décoloration ou de décapage, ainsi que des dispositifs de teinture ou « kits » à plusieurs compartiments.

D'autres caractéristiques, aspects, objets et avantages de l'invention figurant dans la description ci-dessous permettront de mieux cerner l'invention.

Par décapage, on entend au sens de la présente invention destruction totale ou partielle des pigments ou colorants synthétiques présents sur ou dans les fibres kératiniques résultant d'un procédé de coloration directe ou d'oxydation.

Par décoloration, on entend au sens de la présente invention destruction totale ou partielle des pigments naturels présents dans les fibres kératiniques (en particulier eumélanines et phaéomélanines).

La présente invention a donc pour objet une composition oxydante pour fibres kératiniques en particulier fibres kératiniques humaines, et plus particulièrement les cheveux, comprenant dans un milieu cosmétiquement acceptable,
(a) au moins un agent oxydant choisi dans le groupe formé par le peroxyde d'hydrogène et les composés susceptibles de produire par hydrolyse du peroxyde d'hydrogène ou leurs mélanges,
(b) au moins un copolymère à base d'acide 2-acrylamido-2-méthylpropane sulfonique et d'acrylamide, et
(c) au moins un polymère choisi parmi les homopolymères d'acide 2-acrylamido-2-méthylpropane sulfonique réticulés ou les copolymères amphiphiles constitués d'au moins une séquence de motifs acide 2-acrylamido-2-méthylpropane sulfonique, et au moins un motif comportant une partie hydrophobe.

Par partie hydrophobe, on entend au sens de la présente invention une chaîne grasse hydrocarbonée comportant de 6 à 50 atomes de carbone ramifiée ou non, saturée ou insaturée.

Les polymères contenus dans la composition conforme à l'invention sont sous forme libre ou sont neutralisés partiellement ou totalement par une base minérale (soude, potasse, ammoniaque) ou une base organique telle que la mono-, di, ou tri-éthanolamine, un aminométhylpropanediol, la N-méthyl-glucamine, les acides aminés basiques comme l'arginine et la lysine et les mélanges de ces composés.

Le copolymère (b) est formé par de l'acide 2-acrylamido-2-méthylpropane sulfonique et de l'acrylamide. On peut citer comme produits commerciaux les produits commercialisés sous forme d'émulsion inverse sous les références SEPIGEL30J ou SIMULGEL600 par la société SEPPIC.

Les polymères (c) contiennent au moins une séquence de motifs acide 2-acrylamido-2-méthylpropane sulfonique. Ainsi lorsque ces polymères ne sont constitués que par cette séquence, il s'agit d'homopolymères poly(acide 2-acrylamido-2-méthylpropane sulfonique). Le passage du brevet FR 2 753 372 consacré à la description de ces homopolymères est incorporé dans la présente demande.

Selon l'invention, les polymères poly(acide 2-acrylamido-2-méthylpropane sulfonique) sont réticulés, de préférence par le triacrylate de triméthylol propane et comprennent, distribués de façon aléatoire :
- de 90 à 99,9% en poids de motifs de formule générale (1) suivante: 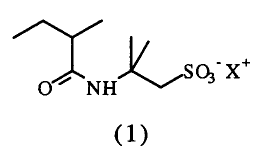 dans laquelle X⁺ désigne un cation, préférentiellement l'ammonium, ou un mélange de cations, au plus 10% mol des cations pouvant être des protons H⁺;
- de 0,01 à 10% en poids de motifs réticulants provenant d'au moins un monomère ayant au moins deux doubles-liaisons oléfiniques ; les proportions en poids étant définies par rapport au poids total du polymère.

De façon préférentielle, l'homopolymère poly(acide 2-acrylamido-2-méthylpropane sulfonique) réticulé comporte un nombre de motifs de formule (1) dans une quantité suffisamment élevée pour obtenir des particules de polymère dont le volume hydrodynamique en solution d'eau présente un rayon allant de 10 à 500 nm et dont la distribution est homogène et unimodale.

Les polymères poly(acide 2-acrylamido-2-méthylpropane sulfonique) réticulés sont présents dans les compositions cosmétiques de l'invention dans des concentrations allant de 0,01 à 10%, et plus particulièrement de 0,05 à 5%, en poids par rapport au poids total de la composition.

Lorsque les polymères (c) contiennent plus d'une séquence de motifs acide 2-acrylamido-2-méthylpropane sulfonique, le copolymère est choisi parmi les copolymères amphiphiles constitués d'au moins une séquence de motifs acide 2-acrylamido-2-méthylpropane sulfonique, et d'au moins un motif comportant une partie hydrophobe. Ces polymères amphiphiles sont décrits dans la demande de brevet FR 2 818 540. Le passage de cette demande consacré à la description de ces polymères amphiphiles est incorporé dans la présente demande.

Les copolymères amphiphiles ont un poids moléculaire en poids allant de 20 000 à 10 000 000, préférentiellement de 50 000 à 8 000 000, plus particulièrement de 100 000 à 7 000 000.

Ceux-ci peuvent être réticulés. Lorsqu'ils le sont, les agents de réticulation peuvent être choisis parmi les composés à polyinsaturation oléfinique couramment utilisés pour la réticulation des polymères obtenus par polymérisation radicalaire. Préférentiellement ces agents sont choisis parmi le méthylène-bis-acrylamide, le méthacrylate d'allyle ou le triacrylate de triméthylol propane (TMPTA). Le taux de réticulation varie de préférence de 0,01 à 10 % en moles et plus particulièrement de 0,2 à2% en moles par rapport au polymère.

Les copolymères amphiphiles comprennent au moins une séquence d'un monomère hydrophobe à insaturation éthylènique comportant au moins une partie hydrophobe allant de 6 à 50 atomes de carbone, préférentiellement de 6 à 22 et plus particulièrement de 12 à 18 atomes de carbone.

Ce monomère hydrophobe à insaturation éthylènique est choisi parmi les acrylates ou les acrylamides de formule (2) suivante : 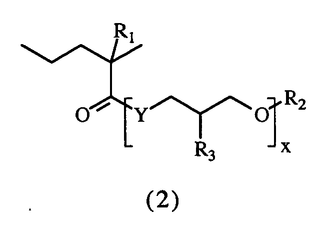 dans laquelle R₁ et R₃, identiques ou différents, désignent un atome d'hydrogène ou un radical alkyle, linéaire ou ramifiée en C₁-C₆, de préférence méthyle ; Y désigne O ou NH ; R₂ désigne un radical hydrocarboné hydrophobe comportant au moins de 6 à 50 atomes de carbone et plus préférentiellement de 6 à 22 atomes de carbone et encore plus préférentiellement de 6 à 18 atomes de carbone; x désigne un nombre de moles d'alkylène et varie de 0 à 100.

Les polymères amphiphiles décrits ci-dessus sont présents dans des concentrations allant de 0,01 à 30 % en poids, plus préférentiellement de 0,1 à 10 % en poids et encore plus préférentiellement de 0,5 à 2 % en poids par rapport au poids total de la composition.

Un autre objet de l'invention est un procédé de teinture d'oxydation des fibres kératiniques humaines et en particulier les cheveux mettant en oeuvre une composition colorante comprenant, dans un support approprié pour la teinture des fibres kératiniques, au moins un précurseur de colorant d'oxydation et une composition oxydante telle que définie ci-dessus. Au moment de l'emploi, la composition colorante est mélangée avec la composition oxydante décrite ; le mélange obtenu est ensuite appliqué sur les fibres kératiniques et laissé poser pendant 3 à 50 minutes environ, de préférence 5 à 30 minutes environ, ensuite suit une étape de rinçage, de lavage au shampooing, à nouveau un rinçage et enfin un séchage. La composition colorante et la composition oxydante décrite peuvent être appliquées séquentiellement, et dans n'importe quel ordre, avec ou sans rinçage intermédiaire.

Un autre objet de la présente invention est un procédé de traitement des fibres kératiniques humaines et en particulier les cheveux, en vue d'obtenir une déformation permanente de ces dernières, en particulier sous la forme de cheveux permanentés, ce procédé comprenant les étapes suivantes.

Une composition réductrice est appliquée sur la matière kératinique à traiter, la fibre kératinique étant mise sous tension mécanique avant, pendant ou après ladite application. Celle-ci se fait mèche par mèche ou globalement.

Il convient de manière classique, de laisser reposer pendant quelques minutes, généralement 5 minutes à une heure, la chevelure sur laquelle a été appliquée la composition réductrice. Ainsi, le réducteur a le temps d'agir sur le cheveu. Cette phase d'attente est effectuée de préférence à une température allant de 35°C à 45°C, en protégeant de préférence également les cheveux par un bonnet.

La fibre kératinique imprégnée de la composition réductrice est éventuellement rincée par une composition aqueuse.

La composition oxydante de l'invention est appliquée sur la fibre kératinique éventuellement rincée, afin de fixer la nouvelle forme imposée aux cheveux. De nouveau, la chevelure traitée est laissée au repos, pendant 3 à 30 minutes, de préférence entre 5 à 15 minutes.

La fibre kératinique subit, à nouveau, un rinçage, généralement à l'eau.

La composition oxydante selon l'invention peut également être utilisée dans un procédé de décoloration ou de décapage des fibres kératiniques humaines et en particulier les cheveux, comprenant les étapes suivantes : une application sur la fibre kératinique de la composition oxydante selon l'invention puis un étape de rinçage de la fibre kératinique ainsi traitée.

Un autre objet de l'invention est un dispositif à 2 compartiments pour la teinture ou pour la déformation permanente ou pour la décoloration des fibres kératiniques en particulier de fibres kératiniques humaines. Un premier compartiment renferme soit une composition colorante, soit une composition réductrice, soit une première composition oxydante, et un second compartiment renferme la composition oxydante définie ci-dessus.

Dans le cadre de la décoloration, la première composition est de préférence une poudre ou une pâte anhydre, contenant au moins un persel.

Les exemples suivants illustrent l'invention sans présenter un caractère limitatif.

### Exemple 1

La demanderesse a réalisé un test comparatif afin de mettre en évidence l'amélioration apportée au niveau de la fluidification lors du mélange de la composition oxydante et de la composition colorante.

La demanderesse a réalisé 3 compositions, 2 conformes à l'art antérieure et l'une conforme à l'invention. (tableau suivant)

| | Formule A conforme à l'art antérieur | Formule B conforme à l'art antérieur | Formule C conforme à l'invention |
|---|---|---|---|
| Eau oxygénée | 6% | 6% | 6% |
| Acide polyacrylamidométhylpropane sulfonique réticulé et partiellement neutralisé à 50% par ammoniaque vendu par Clariant sous la dénomination Hostacerin AMPS | 1,5% | / | 1,5% |
| Copolymère Acrylamide/acrylamido méthylpropane sulfonique sel de sodium en émulsion inverse dans isohexadécane/eau vendu par Seppic sous la dénomination Simulgel 600 | / | 1% | 1% |
| Agent de pH | Qsp pH=3,6 | Qsp pH=3,6 | Qsp pH=3,6 |
| eau | Qsp 100g | Qsp 100g | Qsp 100g |

La stabilité de ces gels oxydants a été étudiée dans le temps. Cette appréciation a été effectuée par un panel de 5 experts.

La composition de formule B est instable. En effet, elle présente une chute de la viscosité après 2 mois à 45°C. Alors que les compositions de formules A et C restent stables.

Le support colorant utilisé pour effectuer les mélanges est le support commercial EXCELLENCE nuance 5 de la société L'ORÉAL. La fluidification a été évaluée par un panel de 5 experts.

Les compositions de formules A et B présentent une fluidification importante dés les premiers instants du mélange.

La composition de formule C ne présente pas de fluidification significative observée.

Ces tests comparatifs montrent clairement l'effet supérieur de l'association des deux polymères, contrairement aux compositions ne comportant qu'un seul polymère.

### Exemple 2

De même, lorsqu'un copolymère amphiphile selon l'invention est associé au polymère acide 2-acrylamido-2-méthylpropane sulfonique, selon la formule D, la fluidification n'est pas observée lors du mélange avec le support colorant commercial EXCELLENCE nuance 5 vendu par la société L'ORÉAL. Cette étude a été évaluée par un panel de 5 experts.

| | Formule D conforme à l'invention |
|---|---|
| Eau oxygénée | 6% |
| Stannate de sodium | 0,04% |
| Pyrrophosphate de sodium | 0,03% |
| alcoolisostéarylique | 2% |
| Copolymère AMPS/méthacrylate de cetearyl éthoxyle(25 EO) 80/20, réticulé par triméthylolpropane triacrylate (TMPTA) vendu par Clariant sous la dénomination Aristoflex HMS | 1% |
| Copolymère Acrylamide/acrylamido méthylpropane sulfonique, sel de sodium en émulsion inverse dans isohexadécane/eau vendu par Seppic sous la dénomination Simulgel 600 | 1 % |
| Agent de pH | Qsp pH=3,5 |
| eau | Qsp 100g |

## Revendications

1. Composition oxydante pour fibres kératiniques en particulier fibres kératiniques humaines, et plus particulièrement les cheveux, comprenant dans un milieu cosmétiquement acceptable,
(a) au moins un agent oxydant choisi dans le groupe formé par le peroxyde d'hydrogène et les composés susceptibles de produire par hydrolyse du peroxyde d'hydrogène ou leurs mélanges,
(b) au moins un copolymère à base d'acide acrylamido-2-méthylpropane sulfonique et d'acrylamide, et
(c) au moins un polymère choisi parmi les homopolymères d'acide 2-acrylamido-2-méthylpropane sulfonique réticulés ou les copolymères amphiphiles constitués d'au moins une séquence de motifs acide 2-acrylamido-2-méthylpropane sulfonique, et au moins un motif comportant une partie hydrophobe.

2. Composition selon la revendication 1 **caractérisée par le fait qu'**elle comprend,
(a) du peroxyde d'hydrogène,
(b) au moins un copolymère à base d'acide acrylamido-2-méthylpropane sulfonique et d'acrylamide , et
(c) au moins un homopolymère d'acide 2-acrylamido-2-méthylpropane sulfonique réticulé.

3. Composition selon la revendication 1 **caractérisée par le fait qu'**elle comprend,
(a) du peroxyde d'hydrogène,
(b) au moins un copolymère à base d'acide acrylamido-2-méthylpropane sulfonique et d'acrylamide, et
(c) au moins un copolymère amphiphile constitué d'au moins une séquence de motifs acide 2-acrylamido-2-méthylpropane sulfonique, et au moins un motif comportant une partie hydrophobe.

4. Composition selon la revendication 3 **caractérisée par le fait qu'**elle comprend, comme copolymères amphiphiles (c) le copolymère acide 2-acrylamido-2-méthylpropane sulfonique /méthacrylate de cétéaryl éthoxyle réticulé par du triacrylate de triméthylolpropane.

5. Composition selon la revendication 2, **caractérisée par le fait que** l'homopolymère (c) poly(acide 2-acrylamido-2-méthylpropane sulfonique) réticulé comprend, distribué de façon aléatoire :
- de 90 à 99,9% en poids de motifs de formule générale (1) suivante : 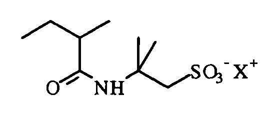 dans laquelle X⁺ désigne un cation ou un mélange de cations, préférentiellement l'ammonium, au plus 10% mol des cations pouvant être des protons H⁺;
- de 0,01 à 10% en poids de motifs réticulants provenant d'au moins un monomère ayant au moins deux doubles-liaisons oléfiniques ; les proportions en poids étant définis par rapport au poids total du polymère.

6. Composition selon la revendication 3 **caractérisée par le fait que** les copolymères amphiphiles ont un poids moléculaire en poids allant de 20 000 à 10 000 000, préférentiellement de 50 000 à 8 000 000, plus particulièrement de 100 000 à 7 000 000.

7. Composition selon l'une des revendications 3 et 6, **caractérisée par le fait que** les copolymères amphiphiles comprennent au moins une séquence de monomères choisis parmi les monomères hydrophobes à insaturation éthylènique comportant au moins une partie hydrophobe ayant de 6 à 50 atomes de carbone, préférentiellement de 6 à 22 et plus particulièrement de 12 à 18 atomes de carbone.

8. Composition selon la revendication 6, **caractérisée par le fait que** le monomère hydrophobe à insaturation éthylènique est choisi parmi les acrylates ou les acrylamides de formule (2) suivante : 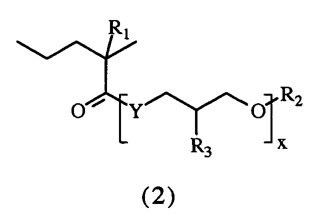 dans laquelle R₁ et R₃, identiques ou différents, désignent un atome d'hydrogène ou un radical alkyle linéaire ou ramifiée en C₁-C₆ (de préférence méthyle) ; Y désigne O ou NH ; R₂ désigne un radical hydrocarboné hydrophobe comportant au moins de 6 à 50 atomes de carbone et plus préférentiellement de 6 à 22 atomes de carbone et encore plus préférentiellement de 6 à 18 atomes de carbone; x désigne un nombre de moles d'alkylène et varie de 0 à 100.

9. Procédé de teinture d'oxydation des fibres kératiniques humaines et en particulier les cheveux mettant en oeuvre
(i)une composition colorante comprenant, dans un support approprié pour la teinture des fibres kératiniques, au moins un précurseur de colorant d'oxydation et
(ii) la composition oxydante telle que définie dans l'une quelconque des revendications de 1 à 8.

10. Procédé de teinture selon la revendication 9 selon lequel:
(i) on mélange, au moment de l'emploi, la composition colorante avec la composition oxydante revendiquée,
(ii) on applique ensuite le mélange obtenu sur les fibres kératiniques,
(iii) on laisse poser pendant 3 à 50 minutes environ, de préférence 5 à 30 minutes environ,
(iv) on effectue ensuite des étapes de rinçage, de lavage au shampooing, à nouveau un rinçage et enfin un séchage.

11. Procédé de teinture selon la revendication 10 selon lequel est appliquée séquentiellement la composition colorante et la composition oxydante revendiquée, dans n'importe quel ordre, avec ou sans rinçage intermédiaire.

12. Procédé de traitement des fibres kératiniques humaines et en particulier les cheveux, en vue d'obtenir une déformation permanente de ces dernières, en particulier sous la forme de cheveux permanentés, ce procédé comprenant les étapes suivantes :
(i) on applique une composition réductrice sur la matière kératinique à traiter, la fibre kératinique étant mise sous tension mécanique avant, pendant ou après la dite application,
(ii) la fibre kératinique est éventuellement rincée
(iii) la composition oxydante telle que définie dans l'une quelconque des revendications de 1 à 8, est appliquée sur la fibre kératinique éventuellement rincée
(iv) la fibre est ensuite rincée

13. Procédé de décoloration ou de décapage des fibres kératiniques humaines et en particulier les cheveux, comprenant les étapes suivantes :
(i) on applique la composition oxydante telle que définie dans l'une quelconque des revendications de 1 à 8 sur la fibre kératinique,
(ii) la fibre traitée est ensuite rincée.

14. Dispositif à 2 compartiments pour la teinture des fibres kératiniques en particulier de fibres kératiniques humaines et plus particulièrement les cheveux, **caractérisé par le fait qu'**un premier compartiment renferme la composition colorante, un second compartiment renferme une composition oxydante telle que définie dans l'une quelconque des revendications 1 à 8.

15. Dispositif à 2 compartiments pour la déformation permanente des fibres kératiniques en particulier de fibres kératiniques humaines et plus particulièrement les cheveux, **caractérisé par le fait qu'**un premier compartiment renferme une composition contenant au moins un réducteur approprié pour la déformation permanente des fibres kératiniques et qu'un second compartiment renferme une composition oxydante telle que définie dans l'une quelconque des revendications 1 à 8.

16. Dispositif à 2 compartiments pour la décoloration des fibres kératiniques en particulier de fibres kératiniques humaines et plus particulièrement les cheveux, **caractérisé par le fait qu'**un premier compartiment renferme une poudre ou une pâte anhydre contenant au moins un persel, et qu'un second compartiment renferme une composition oxydante telle que définie dans l'une quelconque des revendications de 1 à 8.
